Europäisches Patentamt

European Patent Office

Office européen des brevets

(11) Publication number: **0 278 677 B1**

# EUROPEAN PATENT SPECIFICATION

(45) Date of publication of patent specification: **22.07.92**   (51) Int. Cl.⁵: **G01N 1/10**, G01N 1/04

(21) Application number: **88300937.5**

(22) Date of filing: **04.02.88**

(54) Sampling device.

(30) Priority: **11.02.87 GB 8703081**

(43) Date of publication of application:
**17.08.88 Bulletin  88/33**

(45) Publication of the grant of the patent:
**22.07.92 Bulletin  92/30**

(84) Designated Contracting States:
**AT BE CH DE ES FR GB GR IT LI LU NL SE**

(56) References cited:
**EP-A- 0 071 491
DE-A- 3 019 341
FR-A- 2 261 517
NL-A- 8 202 700
US-A- 4 368 272**

**JOURNAL OF VIROLOGICAL METHODS, vol. 12, 1985, pages 31-39, Elsevier Science Publishers B.V.; P.H. BERGER et al.: "Detection of picogram quantities of potyviruses using a dot blot immunobinding assay"**

(73) Proprietor: **SECRETARY OF STATE FOR SCOTLAND
New St. Andrew's House
Edinburgh EH1 3SX Scotland(GB)**

(72) Inventor: **Howell, Peter J., Dr.
8 Traquair Park East
Edinburgh 12 Scotland(GB)**
Inventor: **Rose, D.Gavin.,
50 North Gyle Road
Edinburgh 12 Scotland(GB)**
Inventor: **Mitchell, David H.
305 Lanark Road West
Edinburgh Scotland(GB)**

(74) Representative: **Sheard, Andrew Gregory et al
Kilburn & Strode 30, John Street
London WC1N 2DD(GB)**

## Description

This invention relates to a method of sampling tissue and/or fluids of biological origin, particularly of plant origin, and to a device suitable for use in such a method.

The need to sample plant tissue and/or fluids stems from, among other things, the desirability of being able to diagnose and monitor plant disease. The economic implications of being able accurately and quickly to diagnose and monitor plant diseases need no emphasis.

Many diagnostic or other monitoring tests for plant disease require relatively complex equipment that is not conveniently or practicably transportable in the field. Samples of plant tissue and/or fluid therefore needs to be taken back to the laboratory, where they may undergo conventional biochemical analyses or some of the more recently developed tests such as immunodiagnostic tests using for example monoclonal antibodies or nucleic acid analysis using for example DNA or RNA probes or sequencing methodology.

For the purposes of, for example, laboratory analysis, there is therefore a need for a convenient method of obtaining, securing and transporting small samples of plant sap or other fluid or tissue to the laboratory for such purposes as analysis for plant viruses and other organisms. It is envisaged that an appropriate method could also find application in the sampling of animal tissue and/or fluid.

According to a first aspect of the present invention, there is provided a method of taking a sample of a detectable biological material, the method being characterised by placing biological tissue between a layer which is adsorbent for detectable biological material and a substrate and applying pressure to urge the adsorbent layer and the substrate towards each other, thereby causing detectable biological material to be adsorbed on the adsorbent layer.

Detectable biological material may thus in effect be immobilised by the adsorbent layer, with which it may form an interaction which may be unaffected by subsequent analysis or processing. The sample may be described as being 'captured' by the adsorbent layer. The detectable biological material will often be antigenic (eg proteinaceous) material allowing for subsequent immunological detection. However, other detectable material, for example, nucleic acid, may also be adsorbed. Nucleic acid may be detectable by sequencing and/or hybridisation. Proteins may be analysed by amino acid analysis.

It is very much preferred that a second substrate be provided the other side of the adsorbent layer from the first substrate. Pressure can then be applied to urge the two substrates towards each other, thereby causing the desired adsorption of protein and/or nucleic acid.

More than one layer may be provided: for example, the biological tissue may in use be sandwiched between adsorbent layers.

The combination of the adsorbent layer and the substrate(s) will for convenience be referred to as a "device".

Although it is usual for only small volumes of liquid to be involved in sampling, in view of the fact that detectable biological material may be detected in less than 1 microlitre of, for example, plant sap, the adsorbent layer is preferably also absorbent so that the sample dries rapidly. Rapid drying is useful as it allows the device to be handled with a reduced risk of contamination. The chances of sample degradation by enzymic reaction are also reduced. For the same reasons a small sample size is preferred.

The adsorbent layer may be in the form of a sheet. When there are two substrates, they are preferably hinged together, either at a common edge along some other line. The adsorbent sheet can be retained on one of the substrates by, for example, adhesive, although any suitable retaining means, such as stapling, tacking or pinning, can be used.

When there are two substrates, the surface of at least one of them that faces the other (the inner surface) may be coated with adsorbent material. This arrangement is usually an alternative to having a separate sheet of adsorbent material.

The preferred material for the adsorbent layer is nitrocellulose, which has the ability to adsorb nucleic acids and proteins. It is this property of nitro-cellulose which underlies the "dot blot" method of polyvirus detection described by Berger et al (Virol. Methods **12** (1985) 31-39). However, although the use of nitrocellulose is preferred for its affinity for proteins and nucleic acids, it is not necessarily the only suitable material, as other adsorbent materials may be able to capture sufficient detectable biological material from, for example, plant tissue and/or fluid (such as sap) to enable effective analysis to take place in the laboratory. Examples of other suitable but less preferred adsorbent materials include adsorbent paper (such as blotting paper or filter paper), cotton wool, cellulose wadding and woven or non-woven fabrics, lint and synthetic material (such as nylon) for example in the form of a membrane. In some circumstances the adsorbent layer may be provided by the surface of at least one of the substrates. More than one adsorbent layer may be provided if desired.

When the adsorbent layer is coated on a substrate it may be applied as a liquid solution or suspension. For example, nitrocellulose may be dissolved in an appropriate solvent.

The two substrates are for convenience of identical or similar construction as each other. They will generally be inert, and it is preferred that they be generally less adsorbent than the adsorbent layer, so as to enhance the efficacy of the adsorbent layer. It is also preferred that the substrates be flexible, and to achieve this and other desirable characteristics, it has been found convenient to make the substrates at least partially of plastics material or paper or cardboard coated with a plastics or other inert layer, at least in the region of the adsorbent layer, which in turn, as described above, may be coated on the substrate(s). The substrates are preferably in the form of sheets.

One or both of the surfaces which in use, are in contact with the tissue and/or fluid (that is, the specimen) may be made abrasive or otherwise adapted to assist in crushing.

One of the substrates, for example the one to which an adsorbent sheet is not attached, may be at least partially removable from the device, so that laboratory reagents may during subsequent analysis be readily applied to the adsorbent layer. When the substrate is a sheet of, for example, plastics material or coated paper or cardboard, it may conveniently be perforated so that a portion of it can easily be removed. For even easier accessibility of the adsorbent layer to reagents, both substrates may be removable (at least in part) for example by virtue of being perforated.

If there are two adsorbent layers (which may be coated each onto one substrate), taking the sample will result in two almost identical samples. The two samples may be processed together, or one may be retained as a check test.

The device is preferably provided with data recordal means, for example in the form of an identification label on which can be recorded data relevant to the tissue being sampled. Such data might include the name and address of the person for whom the sample is to be analysed, a serial number, the data, the type(s) of analysis to which the sample is to be submitted and/or possibly an indication of any suspected infection, the causative agent of which is to be looked for on analysis. The data recordal means can conveniently be provided as or on an extension of one or both of the substrates. The two substrates may therefore be fixed together in a region away from the adsorbent layer.

When at least one of the substrates is coated with the adsorbent layer, identification data (such as a serial number) may be provided on a coated and detachable portion of the substrate (or of one or each of the substrates) and repeated in an identification label. This will allow remote processing of the functional portion of the device. After analysis, results can be identified by matching the identification data on the coated and detachable portion with the data on the label. This can lead to a reduced size of the portion subjected to analysis and the possibility of complete immersion of the coated detached area without all the information on the label being subjected to the risk of becoming illegible during the analysis process.

The overall size of the device need not be large. In the case of substantially planar substrates and adsorbent layer, the thickness need only be in the order of 0.5 to 5mm, for example about 1 or 2mm. The length, including an identification label, can vary, for example from 50mm to 200mm. A convenient length is about 110mm. The width may vary from 10 to 50mm, although about 25mm has been found to be convenient. With the convenient proportions just described, the layer of adsorbent material may be about 20 × 15mm, although its size will generally be chosen with regard being had to whatever size of substrates are used.

Although dimensions have been given to illustrate convenient size ranges, they are not absolutely limiting. The lower size limit of the device will generally be that which allows reliable sampling without contamination by either the operator or any device giving a mechanical advantage (for example a pair of pliers, tweezers or forceps) which he may be using to apply pressure to the device. Given this, it is generally preferable to have the device as small as possible both to allow sampling of small organisms and to economise on test reagents. The upper size limit may be determined only by convenience; for example it may be wished to use the device to take a sap sample from the whole (or a large part of the) area of a large leaf.

According to a second aspect of the present invention there is provided a biological tissue and/or fluid sampling device comprising a layer of nitrocellulose sandwiched between two substrates, the nitrocellulose and the substrates being held together in such a way that biological, for example plant, tissue can be removably inserted between the nitrocellulose and at least one of the substrates.

Other preferred features of the second aspect of the invention are as for the first aspect mutatis mutandis.

For a better understanding of the present invention and to show how it may be put into effect, reference will now be made to the accompanying drawing, in which:

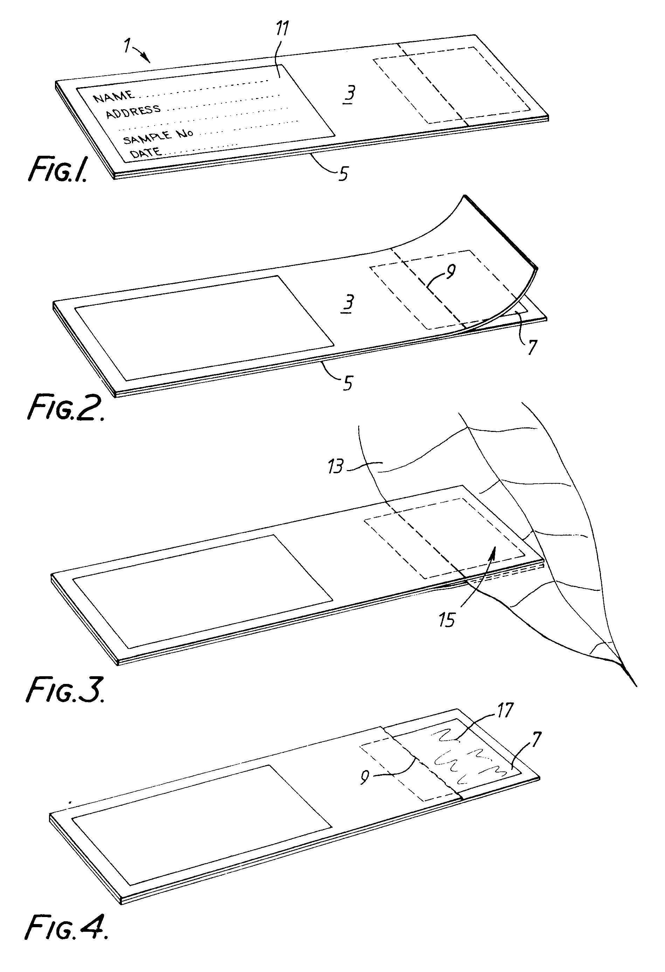

FIGURE 1 is a perspective view of the device in accordance with the invention ready for use;

FIGURE 2 is a perspective view of the same device shown in Figure 1 (with some features omitted for clarity), the substrate being splayed apart to show the absorbent layer in between them;

FIGURE 3 shows the device shown in Figure 1 in use taking a sample from a plant leaf; and

FIGURE 4 shows the same device after the sample has been taken and ready for laboratory processing.

Referring now to the drawings, a plant sampling device 1 in accordance with the invention comprises two thin sheets of inert plastics material. The upper sheet 3 and the lower sheet 5 form inert substrates and are bonded together at their left hand ends for about three quarters of their length. The last 15mm or so at the right hand end are not bonded together, so that they may hinge apart to reveal a nitrocellulose layer 7 which is located between them and bonded to the lower plastics layer 5. (In an alternative embodiment, the nitrocellulose layer may be coated in one or both of the sheets 3 and 5.)

The upper sheet 3 has a line of perforations 9 extending across its width in the region of the nitrocellulose absorbent layer 7, for a purpose that will become clear later.

The upper plastics sheet 3 is provided with an identification label 11, for recording data indicative of the sample to be taken.

The use of the device can be seen in Figure 3. The right hand end is splayed apart to allow its location either side of the margin of a leaf 13 or other plant organ to be sampled. The leaf is thus located between the nitrocellulose adsorbent layer 7 and the upper plastics layer 3. Pressure is then applied to the outside of the upper and lower plastics layers 3 and 5 generally at point 15. By the application of pressure, a small quantity of plant sap (possibly in conjunction with plant tissue) is expressed from the leaf 13 or other organ and captured on the nitrocellulose layer 7. Pressure may be applied by hand or with the aid of a device giving a mechanical advantage, such as a pair of pliers.

The texture of the surface either side of the leaf may be so adapted (for example by being roughened) as to assist in the crushing of the leaf.

If the identification label 11 has not previously been made use of, details of the sample can then be written on the label, which then can be despatched by post or any other mode of transport to a servicing laboratory for the processing of the sample to detect, for example, viruses or other pathogens.

In the laboratory, the upper plastics layer 3 can be torn along the perforation 9 to expose a substantial portion of the nitrocellulose layer 7 and the sap 17 on the nitrocellulose layer. The sap is therefore exposed for chemical analysis, and can be processed in any way and the results recorded and reported in the usual way.

## Claims

1. A method of taking a sample of a detectable biological material, the method being characterised by placing biological tissue (13) between a layer (7) which is adsorbent for said detectable biological material and a substrate (3) and applying pressure to urge the adsorbent layer (7) and the substrate (3) towards each other, thereby causing said detectable biological material to be released from the tissue and adsorbed on the adsorbent layer (7).

2. A method as claimed in claim 1, wherein a second substrate (5) is provided on the other side of the adsorbent layer (7) from the first substrate (3).

3. A method as claimed in claim 1 or 2, wherein the biological tissue (13) is plant tissue and the detectable biological material is plant sap, or a residue of dried plant sap.

4. A method as claimed in claim 2, wherein the two substrates (3), (5) are hinged together.

5. A method as claimed in any one of claims 1 to 4, wherein the adsorbent layer (7) comprises nitrocellulose.

6. A method as claimed in any one or claims 1 to 5, wherein at least one surface which, in use, is in contact with the tissue (13) and/or fluid is abrasive or otherwise adapted to assist in crushing.

7. A sampling device for detectable biological material comprising a layer of nitrocellulose (7) and a substrate (3), the nitrocellulose (7) and the substrate (3) being held together in such a way that biological, for example plant, tissue (13) can be removably inserted between the nitrocellulose (7) and the substrate (3) and that the substrate (3) and the nitrocellulose (7) can be urged towards one another to cause adsorption of the biological material on the nitrocellulose (7).

8. A device as claimed in claim 7, wherein a further substrate (5) is provided on the other side of the nitrocellulose layer (7) from the substrate (3).

9. A device as claimed in claim 7 or claim 8, further comprising data recordal means (11), for example an identification label.

## Revendications

1. Procédé pour prélever un échantillon d'une matière biologique détectable, caractérisé en ce qu'on place un tissu biologique (13) entre une couche (7) qui est adsorbante pour ladite matière biologique détectable et un substrat (3) et on applique une pression pour solliciter la couche adsorbante (7) et le substrat (3) l'un vers l'autre en provoquant ainsi la libération de la matière biologique détectable du tissu et son adsorption sur la couche adsorbante (7).

2. Procédé suivant la revendication 1, dans lequel un second substrat (5) est prévu de l'autre côté de la couche adsorbante (7) par rapport au premier substrat (3).

3. Procédé suivant la revendication 1 ou 2, dans lequel le tissu biologique (13) est un tissu végétal et la matière biologique détectable est du suc de plante ou un résidu de suc de plante séché.

4. Procédé suivant la revendication 2, dans lequel les deux substrats (3, 5) sont articulés l'un à l'autre.

5. Procédé suivant l'une quelconque des revendications 1 à 4, dans lequel la couche adsorbante (7) comprend de la nitrocellulose.

6. Procédé suivant l'une quelconque des revendications 1 à 5, dans lequel au moins une surface qui, en service, est en contact avec le tissu (13) et/ou avec le fluide est abrasive ou rendue autrement apte à favoriser un écrasement.

7. Dispositif de prélèvement d'échantillons de matière biologique détectable comprenant une couche de nitrocellulose (7) et un substrat (3), la nitrocellulose (7) et le substrat (3) étant maintenus assemblés d'une manière telle que le tissu biologique par exemple végétal (13) puisse être inséré de manière amovible entre la nitrocellulose (7) et le substrat (3) et que le substrat (3) et la nitrocellulose (7) puissent être sollicités l'un vers l'autre pour provoquer l'adsorption de la matière biologique sur la nitrocellulose (7).

8. Dispositif suivant la revendication 7, dans lequel un autre substrat (5) est prévu de l'autre côté de la couche de nitrocellulose (7) par rapport au substrat (3).

9. Dispositif suivant la revendication 7 ou 8, comprenant, en outre, des moyens d'enregistrement de données (11), par exemple une étiquette d'identification.

**Patentansprüche**

1. Verfahren zur Probenentnahme eines nachweisbaren biologischen Materials, **dadurch gekennzeichnet,** daß biologisches Gewebe (13) zwischen eine Schicht (7), die für das nachweisbare biologische Material adsorbierend ist, und ein Substrat (3) angeordnet und Druck aufgebracht wird, so daß die adsorbierende Schicht (7) und das Substrat (3) zusammengepreßt werden, wodurch das nachweisbare biologische Material vom Gewebe freigesetzt und von der adsorbierenden Schicht (7) adsorbiert wird.

2. Verfahren nach Anspruch 1, wobei ein zweites Substrat (5) auf der anderen Seite der adsorbierenden Schicht (7) bezogen auf das erste Substrat (3) bereitgestellt wird.

3. Verfahren nach Anspruch 1 oder 2, wobei das biologische Gewebe (13) ein Pflanzengewebe und das nachweisbare biologische Material Pflanzensaft oder ein Überrest von getrocknetem Pflanzensaft ist.

4. Verfahren nach Anspruch 2, wobei die beiden Substrate (3), (5) aneinander angelenkt sind.

5. Verfahren nach einem der Ansprüche 1 bis 4, wobei die adsorbierende Schicht (7) Nitrocellulose enthält.

6. Verfahren nach einem der Ansprüche 1 bis 5, wobei mindestens eine Oberfläche, die bei Gebrauch mit dem Gewebe (13) und/oder der Flüssigkeit in Kontakt ist, abreibend ist oder auf eine andere Art das Zusammenpressen fördert.

7. Vorrichtung zur Probenentnahme für nachweisbares biologisches Material, die eine Nitrocelluloseschicht (7) und ein Substrat (3) enthält, wobei die Nitrocellulose (7) und das Substrat (3) derart zusammengehalten werden, daß biologisches Gewebe (13), z.B. eine Pflanze, auswechselbar zwischen die Nitrocellulose (7) und das Substrat (3) eingefügt werden kann, und daß das Substrat (3) und die Nitrocellulose (7) zusammengepreßt werden können, um die Adsorption des biologischen Materials an die Nitrocellulose (7) zu bewirken.

8. Vorrichtung nach Anspruch 7, bei der ein weiteres Substrat (5) auf der anderen Seite der Nitrocelluloseschicht (7) bezogen auf das Substrat (3) bereitgestellt wird.

**9.** Vorrichtung nach Anspruch 7 oder 8, die ferner ein Mittel (11) zum Erfassen von Daten, z.B. eine Identifizierungsmarkierung, enthält.

Fig.1.

Fig.2.

Fig.3.

Fig.4.